**Europäisches Patentamt**

**European Patent Office**

**Office européen des brevets**

(19)

(11) Veröffentlichungsnummer: **0 135 735**
**B1**

(12) **EUROPÄISCHE PATENTSCHRIFT**

(45) Veröffentlichungstag der Patentschrift:
**18.05.88**

(21) Anmeldenummer: **84109215.8**

(22) Anmeldetag: **03.08.84**

(51) Int. Cl.⁴: **A 61 N 1/39**, A 61 N 1/04

(54) Elektrische Schockelektrodenschaltung für einen Defibrillator.

(30) Priorität: **03.08.83 US 519843**

(43) Veröffentlichungstag der Anmeldung:
**03.04.85 Patentblatt 85/14**

(45) Bekanntmachung des Hinweises auf die Patenterteilung:
**18.05.88 Patentblatt 88/20**

(84) Benannte Vertragsstaaten:
**AT CH DE FR GB IT LI**

(56) Entgegenhaltungen:
**DE-A-2 811 325**
**GB-A-1 298 189**
**US-A-3 196 877**
**US-A-3 389 703**
**US-A-3 942 533**

(73) Patentinhaber: **GS Elektromed. Geräte Günter Stemple, Thorstrasse 13, D-8912 Kaufering (DE)**

(72) Erfinder: **Stemple, Günter, Dipl.- Ing., Thorstrasse 13, D-8912 Kaufering (DE)**

(74) Vertreter: **Nöth, Heinz, Dipl.- Phys., Patentanwälte Pfenning, Meinig & Partner Mozartstrasse 17, D-8000 München 2 (DE)**

EP 0 135 735 B1

## Beschreibung

Die Erfindung betrifft eine elektrische Schockelektrodenschaltung für einen Defibrillator mit zwei Schockelektroden die jeweils einen Bedienungshandgriff und eine Anwendungselektrode, der über eine Entladungsleitung die Energie eines Energiespeichers (Kondensator) der Defibrillatorschaltung zugeführt wird.

Im Energiespeicher der Defibrillatorschaltung lassen sich abgestufte Energiemengen abspeichern und entsprechend abgestufte Energiemengen (Energiedosierung) in Abhängigkeit vom Patienten und der gewünschten Behandlungsart abgeben. Die abgegebenen Energiemengen für externe Defibrillation können zwischen 20 und 320 Joule liegen.

Defibrillatorgeräte sollen leicht transportabel sein, und es wird daher eine offene Bauweise bevorzugt. Bei dieser Bauweise sind die Defibrillatorelektroden gegen Nässe und Verschmutzung von außen ungeschützt. Ferner wird zur Kontaktierung der Anwendungselektroden häufig ein leitendes Gel verwendet, das auf die Elektrodenoberfläche aufgestrichen wird. Die Gefahr der Bildung elektrischer Kriechwege zwischen der Anwendungselektrode und dem Handgriff erhöht sich hierdurch.

Aufgabe der Erfindung ist es daher, eine elektrische Schockelektrodenschaltung für einen Defibrillator zu schaffen, durch die ein optimaler Berührungsschutz an den Schockelektroden erzielt wird.

Diese Aufgabe wird erfindungsgemäß bei der elektrischen Schockelektrodenschaltung der eingangs genannten Art dadurch gelöst, daß zwischen der Anwendungselektrode und dem Bedienungshandgriff jeder der beiden Schockelektroden eine ring-oder scheibenförmige Schutzelektrode vorgesehen ist, und die Schutzelektroden der beiden Schockelektroden elektrisch leitend verbunden sind.

Die beiden Schutzelektroden können über eine Steuerleitung miteinander verbunden sein, wobei in diese Steuerleitung Schalter eingebaut sind.

Für das Anlegen an einen Herzmuskel können die Schockelektroden paddel- oder löffelförmig ausgebildet sein.

Die elektrische Schockelektrodenschaltung kann bevorzugt Anwendung finden bei einem Defibrillator, dessen Schaltung in der DE-OS-3 229 134 beschrieben ist.

Durch die Erfindung wird wegen des optimalen Berührungsschutzes an den Defibrillatorelektroden eine einwandfreie Handhabung unter Berücksichtigung wichtiger Sicherheitsfunktionen gewährleistet.

Anhand der beiliegenden Figuren, die Ausführungsbeispiele der Erfindung darstellen, wird die Erfindung noch näher erläutert. Es zeigen:

Fig. 1 bis 4 Ausführungsformen von Defibrillatorelektroden und
Fig. 5 und 6 Ausführungsformen für den im Defibrillator verwendeten Energiespeicher.

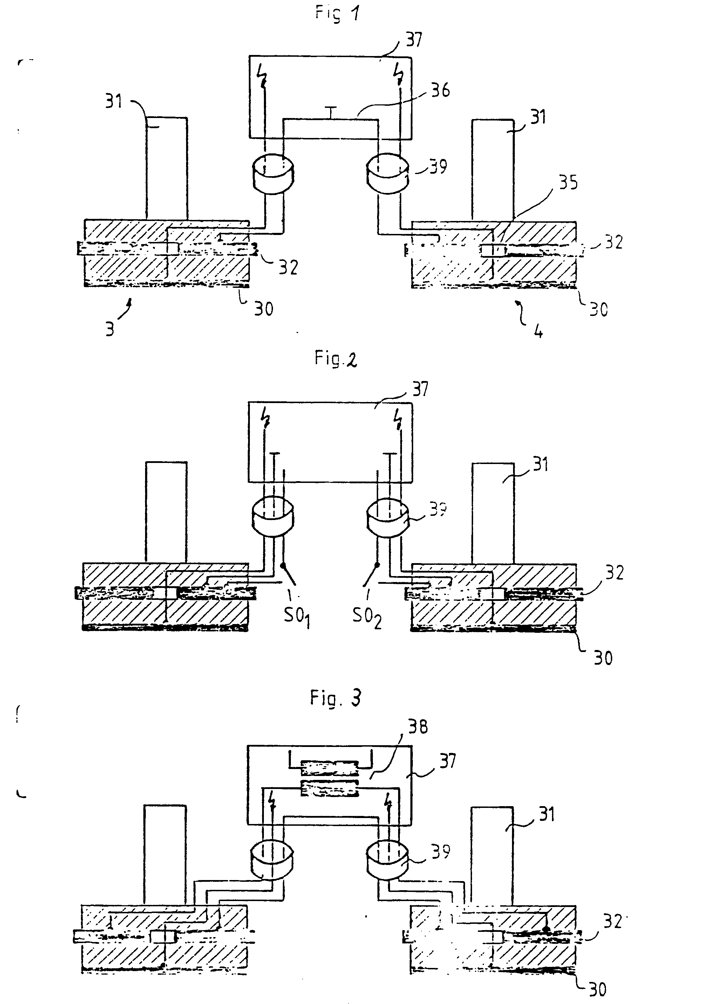

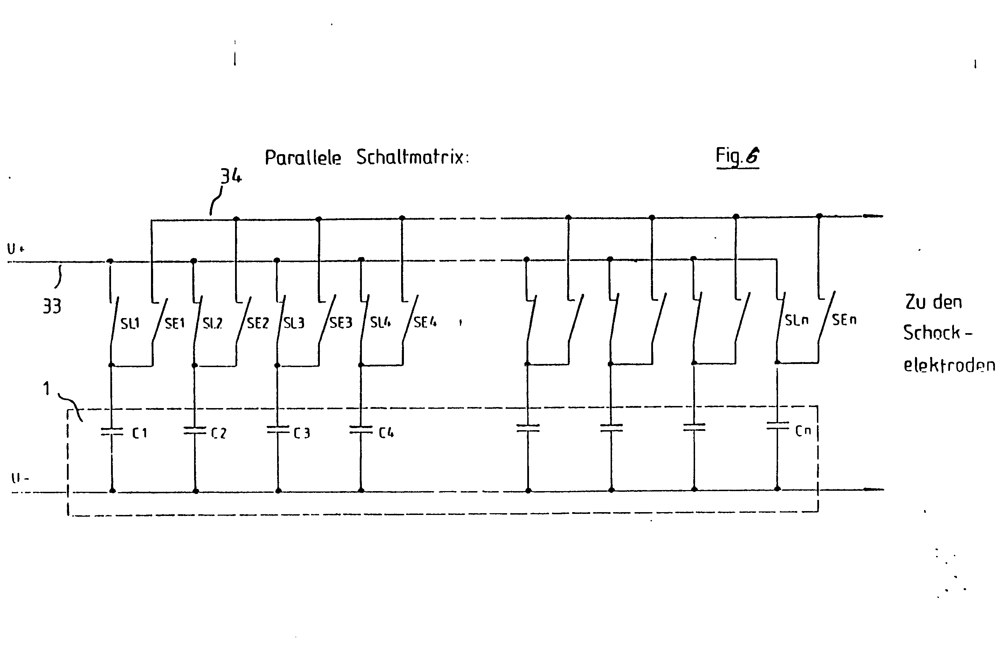

Eine in den Figuren 1 bis 3 gezeigte elektrische Schaltung 37 dient zur Erzeugung von Energieimpulsen an zwei Schockelektroden 3, 4 eines Defibrillators (wie er z. B. in der DE-OS-3 229 134 beschrieben ist), mit einem Energiespeicher (z. B. wie in Fig. 6 und 7), dessen Energie über einen geschlossenen Arbeitsschalter an die Schockelektroden 3, 4 bei Schockbehandlung eines Patienten impulsartig abgeführt wird.

Diese Schockelektroden 3 und 4 besitzen einen Handgriff 31 und eine Anwendungselektrode 30, über die die Energie des Energiespeichers 1 bei der Defibrillation abgegeben wird. Zwischen dem Bedienungshandgriff 31 und der Anwendungselektrode 30 befindet sich eine Schutzelektrode 32, welche scheiben- oder ringförmig ausgebildet sein kann und die zumindest eine um die äußere Umfangsfläche der Defibrillationselektrode umlaufende Fläche besitzt. Bei den dargestellten Ausführungsbeispielen steht die Schutzelektrode 32 über die äußere Umfangsfläche mit einem umlaufenden Randteil über. Die Versorgungsleitung für die Anwendungselektroden 30 ist durch eine Durchführung 35 in der Schutzelektrode 32 hindurchgeführt. Wie aus den Figuren zu ersehen ist, sind die Schutzelektroden 32 der beiden Defibrillatorelektroden über eine Leitung 36 miteinander verbunden. Diese Leitung 36 kann über das Defibrillatorgerät 37 führen. Zur Überwachung der elektrisch leitenden Verbindung zwischen den beiden Schutzelektroden 32 kann die Verbindung über z. B. eine Steuerleitung erfolgen. Hierzu können, wie in der Fig. 2 dargestellt ist, zusätzliche Schalter SO1 und SO2 vorgesehen sein. Beim Ausführungsbeispiel der Fig. 3 erfolgt die Überwachung der Verbindung der beiden Schutzelektroden 32 über einen Koppler 38 und zwei Drahtleitungen.

Die spezielle Ausbildung der Defibrillatorelektroden mit Schutzelektroden 32 dient zur Vermeidung der Gefahr von elektrischen Schlägen für die Bedienungsperson aufgrund leitender Oberflächenverschmutzung der Defibrillatorelektroden. Defibrillatorgeräte sollen leicht transportabel sein, und es wird daher eine offene Bauweise bevorzugt. Bei dieser Bauweise sind die Defibrillatorelektroden gegen Nässe und Verschmutzung von außen nicht geschützt. Ferner wird zur Kontaktierung der Anwendungselektroden häufig ein leitendes Gel verwendet, das auf die Elektrodenoberfläche aufgestrichen wird. Auch hierdurch wird die Gefahr der Bildung elektrischer Kriechwege zwischen der Anwendungselektrode 30 und dem Handgriff 31 erhöht. Um nun die Bedienungsperson absolut vor solchen Kriechströmen zu schützen, wird die Schutzelektrode 32 zwischen dem Handgriff 31 und der Anwendungselektrode 30 vorgesehen.

Wie aus den Figuren 1 bis 3 zu ersehen ist, können die Zuleitungen für die Anwendungselektrode 30 und die Schutzelektrode 32 in gemeinsamen Kabeln 39 untergebracht sein.

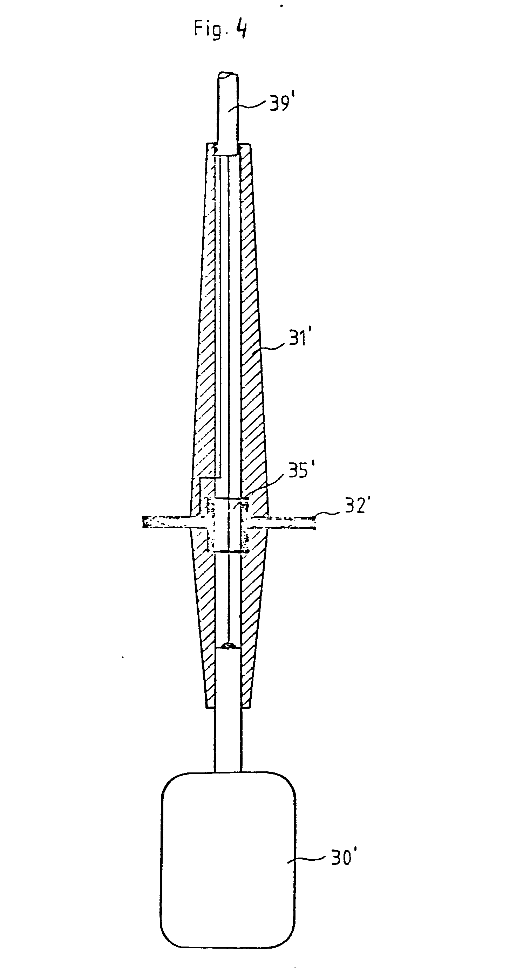

Die Fig. 4 zeigt eine paddel- oder löffelförmige Elektrode, welche direkt an den Herzmuskel angelegt werden kann. Diese Elektrode besitzt eine Anwendungselektrode 30' in Löffel- bzw. Paddelform, welche zur internen Anwendung bei der Defibrillation an den Herzmuskel angesetzt wird. Im stielartigen Bedienungshandgriff 31' ist eine umlaufende Schutzelektrode 32' vorgesehen, die für die Versorgungsleitung eine Durchführung 35' aufweist. In einem gemeinsammen Kabel 39' werden sowohl die Versorgungsleitung für die Anwendungselektrode 30' als auch die mit der Schutzelektrode 32' verbundene Leitung vom stielartigen Bedienungshandgriff 31' weggeführt. Die Verschaltung dieser Leitungen kann in der gleichen Weise erfolgen wie bei den Ausführungsbeispielen der Figuren 1 bis 3 gezeigt ist.

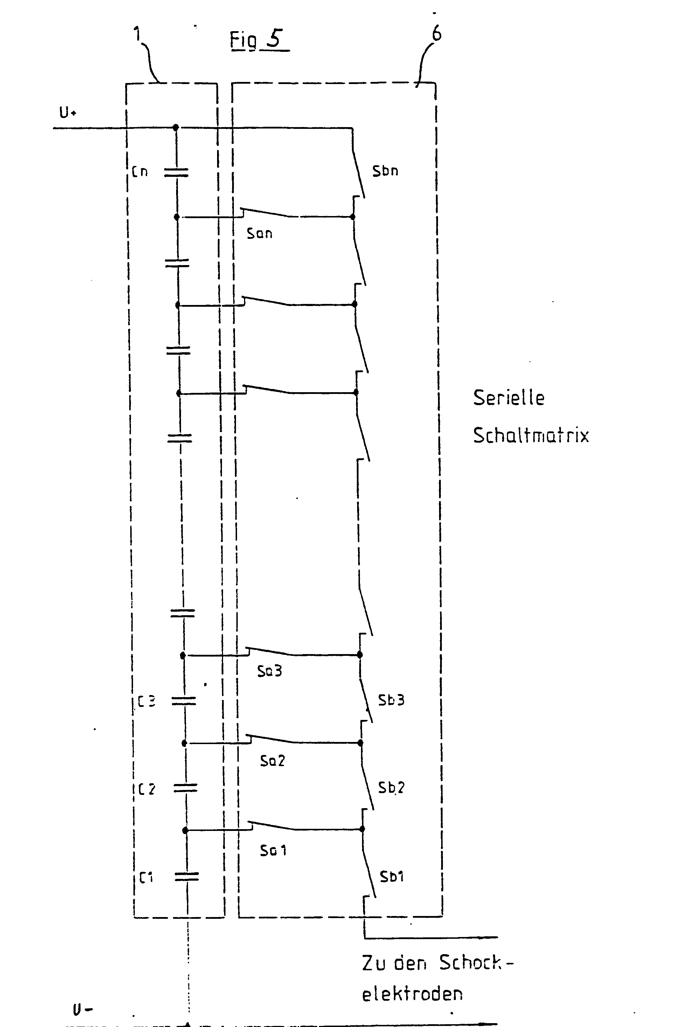

In der Fig. 5 ist ein Ausführungsbeispiel für einen Energiespeicher 1 und einen Arbeitsschalter 6 der Defibrillatorschaltung 37 dargestellt. Der Energiespeicher 1 besteht aus mehreren in Reihe geschalteten Kondensatoren $C_1 ... C_n$. Der Arbeitsschalter 6 besitzt mehrere ebenfalls in Reihe geschaltete Schalter $Sb_1$. $..SB_n$, wobei die Verbindungspunkte jeweils zweier Kondensatoren C1 und C2 ...$C_n$-1 und $C_n$ mit den Verbindungspunkten zweier Schalter $Sb_1$ und $Sb_2$ ...$Sb_n$- 1 und $Sb_n$ verbunden sind über weitere Schalter $Sa_1 ... Sa_n$.

Die in Reihe geschalteten Kondensatoren werden alle auf gleichen Spannungspegel geladen. Das Entladen der Kondensatoren erfolgt nacheinander in genau festgelegten Zeitabschnitten. Hierzu werden nacheinander in diesen festgelegten Zeitabschnitten die Schalter $Sb_1 ... Sb_n$ geschlossen. Der jeweilige Schalter $Sa_1 ... Sa_n$, über welchen die vorhergehende Entladung eines jeweiligen Kondensators erfolgte, wird dabei geöffnet. Bei Beginn der Entladung wird zunächst der Kondensator $C_1$ entladen über den geschlossenen Schalter $Sa_1$ und den geschlossenen Schalter $Sb_1$. Die Schalter $Sb_2 ... Sb_n$ sind geöffnet. Zur Entladung des Kondensators $C_2$ wird der Schalter $Sb_2$ geschlossen und der Schalter $Sa_1$ geöffnet. Der Schalter $Sb_1$ bleibt geschlossen. Auch der Schalter $Sa_2$ ist geschlossen. Das heißt, die Entladung des Kondensators $C_2$ erfolgt über den Schalter $Sa_2$, $Sb_2$, $Sb_1$. In der gleichen Weise erfolgt die Entladung der restlichen Kondensatoren $C_3 ... C_n$. Bevor die Entladung der Kondensatoren beginnt, sind die Schalter $Sb_1$. $..Sb_n$ geöffnet. Die Schalter $Sa_1 ... Sa_n$ können geschlossen sein.

Durch dieses zeitliche nacheinanderfolgende Aufstocken der einzelnen Kondensatorspannungen bei gleichzeitiger Entladung über die Schockelektroden läßt sich eine individuell formbare Entladekurve erzeugen.

Für die Schalter $Sa_1 ... Sa_n$ und $Sb_1 ... Sb_n$ können Dioden, Transistoren, Thyristoren und dgl. verwendet werden.

In der Fig. 6 ist ein weiteres Ausführungsbeispiel für den Energiespeicher 1 dargestellt. bei diesem Ausführungsbeispiel besteht der Energiespeicher aus parallel geschalteten Kondensatoren $C_1 ... C_n$. Die Aufladung der Kondensatoren erfolgt über eine Ladeleitung 33 bei mit den Kondensatoren $C_1 ... C_n$ jeweils in Reihe geschalteten Schaltern $SL_1$. $..SL_n$. Die Entladung erfolgt über eine Entladeleitung 34, wobei die Schalter $SL_1 ... SL_n$ geöffnet und die zu den Kondensatoren $C_1 ... C_n$ jeweils in Reihe liegenden Schalter $SE_1 ... SE_n$ nacheinander in bestimmten Zeitabständen geschlossen werden. Auch hier erfolgt durch das zeitlich nacheinanderfolgende Aufstocken der einzelnen Kondensatorspannungen bei gleichzeitiger Entladung über die Schockelektroden die Bildung einer individuell erwünschten Entladekurve. Beim Ausführungsbeispiel der Fig. 6 übernehmen die Schalter $SE_1 ... SE_n$ die Funktion des Arbeitsschalters 6. Auch für die Schalter $SL_1$. $..SL_n$ und $SE_1 ... SE_n$ eignen sich Dioden, Transistoren, Thyristoren und dgl.

**Patentansprüche**

1. Elektrische Schockelektrodenschaltung für einen Defibrillator mit zwei Schockelektroden (3, 4), die jeweils einen Bedienungshandgriff (31, 31') und eine Anwendungselektrode (30, 30'), der über eine Entladungsleitung (34) die Energie eines Energiespeichers (1) der Defibrillatorschaltung (37) zugeführt wird, aufweisen,
dadurch gekennzeichnet,
daß zwischen der Anwendungselektrode (30, 30') und dem Bedienungshandgriff (31, 31') jeder der beiden Schockelektroden (3 bzw. 4) eine ring- oder scheibenförmige Schutzelektrode (32) vorgesehen ist,
und die Schutzelektroden (32, 32') der beiden Schockelektroden (4 bzw. 3) elektrisch leitend (Leitung 36) verbunden sind.

2. Schockelektrodenschaltung nach Anspruch 1, dadurch gekennzeichnet,
daß die beiden Schutzelektroden (32) über eine Steuerleitung miteinander verbunden sind.

3. Schockelektrdenschaltung nach Anspruch 1 oder 2, dadurch gekennzeichnet,
daß der Energiespeicher (1) aus in Reihe oder parallel geschalteten Kondensatoren ($C_1$, $C_2$ ... $C_n$) besteht, die alle auf gelichen Spannungspegel geladen sind und nacheinander mittels Schaltern ($SA_1 ... SA_n$ und $SB_1 ... SB_n$) in bestimmten Zeitabschnitten entladen werden.

4. Schockelektrodenschaltung nach einem der Ansrüche 1 bis 3 dadurch gekennzeichnet,

daß die Schokelektroden (3, 4) paddel- oder löffelförmig ausgebildet sind.

5. Schockelektrodenschaltung nach einem der Ansprüche 1 bis 4, dadurch gekennzeichnet, daß die Schutzelektrode (32, 32') zumindest eine um die äußere Umfangsfläche der Schockelektrode (3 bzw. 4) umlaufende Fläche besitzt.

6. Schockelektrodenschaltung nach einem der Ansprüche 1 bis 5, dadurch gekenzeichnet,

daß die Schutzelektrode (32, 32') mit einem umlaufenden Randteil über die äußere Umfangsfläche der Schockelektrode (3 bzw. 4) übersteht.

7. Schockelektrodenschaltung nach Anspruch 2, dadurch gekennzeichnet,

daß in die Steuerleitungen Schalter (SO$_1$, SO$_2$) geschaltet sind.

**Claims**

1. An electric shock electrode circuit for a defibrillator comprising two shock electrodes (3, 4) which each have an operating handle (31, 31') and an application electrode (30, 30') which is supplied with the energy of an energy storage means (1) of the defibrillator circuit (37) by way of a discharge line (34), characterised in that provided between the application electrode (30, 30') and the operating handle (31, 31') of each of the two shock electrodes (3 and 4 respectively) is a protective electrode (32) in the form of a ring or disc, and the protective electrodes (32, 32') of the two shock electrodes (4 and 3 respectively) are electrically conductively (line 36) connected.

2. A shock electrode circuit according to claim 1 characterised in that the two protective electrodes (32) arce connected together by way of a control line.

3. A shock electrode circuit according to claim 1 or claim 2 characterised in that the energy storage means (1) comprises capacitors (C1, C2...C$_n$) which are connected in series or parallel and which are all charged to the same voltage level and which are successively discharged by means of switches (SA$_1$ ...SA$_n$ and SB$_1$ ...SB$_n$ ) at certain intervals of time.

4. A shock electrode circuit according to one of claims 1 to 3 characterised in that the shock electrodes (3, 4) are of a paddle-like or spoon-like configuration.

5. A shock electrode circuit according to one of claims 1 to 4 characterised in that the protective electrode (32, 32') has at least one surface extending around the outer peripheral surface of the shock electrode (3 or 4 respectively).

6. A shock electrode circuit according to one of claims 1 to 5 characterised in that the protective electrode (32, 32') projects with a peripherally extending edge portion beyond the outer peripheral surface of the shcck electrcde (3 or 4 respectively).

7. A shock electrode circuit according to claim 2 characterised in that switches (SO$_1$, SO$_2$) are connected into the control lines.

**Revendications**

1. Circuit électrique d'électrodes de choc pour un défibrillateur avec deux électrodes de choc (3, 4) comportant chacune une poignée de manoeuvre (31, 31'), et avec une électrode d'application (30, 30') qui reçoit l'énergie d'un accumulateur d'énergie (1) du circuit de défibrillateur (37) par l'intermédiaire d'une ligne de décharge (34),

caractérisé en ce qu'une électrode de protection (32) en forme d'anneau ou de disque est prévue entre l'électrode d'application (30, 30') et la poignée de manoeuvre (31, 31') de chacune des deux électrodes de choc (3 ou 4), et que les électrodes de protection (32, 32') des deux électrodes de choc (4 ou 3) sont reliées électriquement (ligne 36).

2. Circuit d'électrodes de choc selon la revendication 1, caractérisé en ce que les deux électrodes de protection (32) sont reliées par l'intermédiaire d'une ligne pilote.

3. Circuit d'électrodes de choc selon l'une des revendications 1 ou 2, caractérisé en ce que l'accumulateur d'énergie (1) se compose de condensateurs (C$_1$, C$_2$ ... C$_n$) montés en série ou en parallèle qui sont tous chargés au même niveau de tension et déchargés successivement, à des intervalles déterminés, à l'aide d'interrupteurs (SA$_1$ ... SA$_n$ et SB$_1$ ... SB$_n$).

4. Circuit d'électrodes de choc selon l'une quelconque des revendications 1 à 3, caractérisé en ce que les électrodes de choc (3, 4) sont conformées en palettes ou en cuillères.

5. Circuit d'électrodes de choc selon l'une quelconque des revendications 1 à 4, caractérisé en ce que l'électrode de protection (32, 32') comprend au moins une surface qui entoure la surface périphérique extérieure de l'électrode de choc (3 ou 4).

6. Circuit d'électrodes de choc selon l'une quelconque des revendications 1 à 5, caractérisé en ce que l'électrode de protection (32, 32') dépasse avec un bord périphérique de la surface périphérique extérieure de l'électrode de choc (3 ou 4).

7. Circuit d'électrodes de choc selon la revendication 2, caractérisé en ce que les lignes pilotes (SO$_1$, SO$_2$) sont commutées.

Fig 1

Fig. 2

Fig. 3

1

Fig. 4

39'

31'

35'

32'

30'

0 135 735

Fig 5

Serielle Schaltmatrix

Zu den Schock-elektroden

Parallele Schaltmatrix:                                    Fig.6

34

U+

33          SL1  SE1  SL2  SE2  SL3  SE3  SL4  SE4          SLn  SEn

1

        C1      C2      C3      C4                          Cn

U-

Zu den
Schockelektroden

0 135 735